# EUROPEAN PATENT SPECIFICATION

(11) **EP 3 970 619 B1**
(45) Date of publication and mention of the grant of the patent: **07.06.2023**
(21) Application number: 20197137.1
(22) Date of filing: 21.09.2020
(51) Int. Cl.: A61B 6/00, G16H 40/63, G16H 30/40

(54) **METHOD TO IMPROVE A RADIOGRAPHY ACQUISITION WORKFLOW**
VERFAHREN ZUR VERBESSERUNG DES ARBEITSABLAUFS EINER RÖNTGENAUFNAHME
PROCÉDÉ POUR AMÉLIORER UN FLUX DE TRAVAIL D'ACQUISITION DE RADIOGRAPHIE

(43) Date of publication of application: 23.03.2022
(73) Proprietor: AGFA NV, 2640 Mortsel (BE)
(72) Inventor: BEHIELS, Gert, 2640 Mortsel (BE)
(74) Representative: Verbrugghe, Anne Marie L.

(56) References cited:
- DE-A1-102012 215 496
- US-A1- 2015 085 971
- US-A1- 2016 073 987
- RIZA ALP GULER ET AL: "DensePose: Dense Human Pose Estimation in the Wild", 2018 IEEE/CVF CONFERENCE ON COMPUTER VISION AND PATTERN RECOGNITION, 1 February 2018 (2018-02-01), pages 7297-7306, XP055648964, https://arxiv.org DOI: 10.1109/CVPR.2018.00762

## Description

### Technical Field

The present invention relates generally to a method to improve a medical imaging workflow. It may be related more specifically to a radiography workflow.

### Background of the invention

In preparation of any imaging examination of a patient, a number of steps have to be performed in order to eventually obtain the intended configuration and arrangement of the combination of the device and the patient. Typically, the operator follows a predefined protocol that defines an acquisition order for the different images to be acquired for a certain radiography study. A worklist is presented to the operator that defines the succession of the different images to be acquired. In most cases the order of the acquisitions is pre-programmed. The operator will be forced to respect the acquisition order as presented in the worklist, unless he can overrule the sequence order by manually altering the order for the study. This, however, implies the execution of additional steps.

The overhead associated with the manual interventions that need to be performed by an operator may impair the effectiveness of the utilisation of the medical imaging modality equipment. The medical industry has always been searching for improvements to the techniques and procedures which are involved in these preparative steps, and this in order to relief the operator (which is in most cases a radiographer) as much as possible from the repetitive and burdensome tasks.

As a first step of an imaging acquisition procedure, the patient will need to be positioned in or on the system. This positioning step is carried out with a prime focus on an optimal positioning of the region of interest (that is located somewhere in the patient) with respect to the position of the imaging detector(s) and the imaging source. But at the same time, attention is given to the comfort of the posture that the patient has to assume for the duration of the medical image acquisition itself, making sure that the patient can sustain his position sufficiently long in comfort.

Bringing the patient into the desired position may involve a number of preparative steps such as for instance the lowering of the surface of a positioning table such that the patient can initially mount the table top in a sitting position and then move into a horizontal lying position. In another example, the operator will assist the patient to assume a standing position at the correct distance from the surface of a wall-stand detector, thereby ensuring that his arms are not obstructing the imaging detector.

As a second step, the active components (the radiation source and imaging detector) of the imaging modality are positioned close to or around the region of interest within the patients' body. This configuration step serves to fine-tune the positioning of the active components with respect to the patient, and will ensure that certain distances between the patient and the components are optimized or at least respected in order to ensure an acquisition that meets the quality criteria. This step will in most of the cases involve the manipulation by the operator of either the detector and the imaging source, or both. This second step is then repeated for each successive image in a series or study.

It is to be noted that for each consecutive image acquisition, a manipulation of the system by the operator is required in most cases. This manipulation involves adjustments of the imaging device and/or a repositioning of the patient which requires the full attention of the operator. It would therefore be desirable if in some cases it would be left to the operator to be able to decide on the actual execution sequence of the images to be taken, rather than to have to follow a strictly imposed order through a worklist.

In case that an operator would like to re-order the execution sequence to acquire the ordered images for a patient, he typically has to alter the worklist sequence at the console of the modality workstation. This operation thus requires an additional step to be performed by the operator. It would be desirable that this additional step would not be necessary.

### Summary of invention

The present invention provides a method to improve a radiography acquisition workflow for an acquisition study for a patient on a radiography system, wherein said acquisition study comprises at least one ordered image in at least one image series for which an acquisition protocol is defined, said acquisition protocol comprising a target structure, a patient positioning instruction and an acquisition geometry, the method comprising the steps of displaying said at least one ordered image as an item on an order list on a display means, positioning said patient on said radiography system according to said patient positioning instructions of said acquisition protocol of one of said ordered images, positioning said radiography system according to said acquisition geometry of said acquisition protocol of said one of said ordered images, capturing a reference image from a camera device, said reference image comprising a beam entry area on a surface of said patient defined by said acquisition protocol for said ordered image, identifying a body part and a location of said beam entry area on said body part on said reference image by means of a body part identification algorithm, matching said identified body part to a body part comprising said target structure defined in the acquisition protocol of any of the ordered images in said acquisition study, and matching said identified beam entry area to a beam entry area corresponding to an acquisition geometry and a patient positioning that is defined in the acquisition protocol of any of the ordered images in said acquisition study, displaying said ordered image as a marked item in said order list on said display means, in case of a positive match for said body part and said beam area.

In the context of this invention, a patient study has to be understood as a collection of exams or procedures that are executed or performed on the same or different imaging modality, but for the same patient. A study for a trauma patient -for instance- may comprise a series of projection radiography images and a series or multiple series of CT scans.

A series has a lower hierarchy than a study and should be understood as a grouped collection of images performed on the same imaging modality, and relating to the same region of interest or targeted structure of the patient. One study may thus comprise many series. In its own turn, a series comprises a set of (at least 1) images that are related to the same region of interest of the patient.

For quality reasons, it is an established practice to perform medical imaging studies according to standardized protocols. These so-called acquisition protocols are ultimately designed to create consistency in image acquisition quality across the department and are intended to deliver optimal diagnostic information to the radiologist. An acquisition protocol is an elaborate set of image acquisition specifications and is defined to cover the majority of diagnostic needs. They are typically defined on the basis of the targeted organ or structure (such as for instance: skull, pelvis, hand, lungs, abdomen or alike) and provide instructions for positioning the patient and the projection (or projections) to be used for the acquisition. Some examples of patient positioning are "supine" (lying the back), "prone" (lying on the chest), "lateral" or "upright". The projection or radiographic projection describes the path that the X-ray beam follows through the patient's body from the entrance to the exit. The term "projection" is sometimes explained by the term "angulation" of the modality system, hinting at the inclination angle of the X-ray tube gantry with respect to the patient. Some examples of projections are: AP (anteriori-to-posteriori), PA, lateral, oblique ...

A specific radiographic projection can be achieved by applying an acquisition geometry to a patient that is positioned in certain way. So in order to fully define a radiographic projection for an image acquisition, patient positioning instructions and the acquisition geometry need to be defined.

For consistency reasons, it is important that identical studies performed on different patients are acquired in the same way, allowing the radiologists to compare the studies and acquire experience along the way. For this purpose will the operators be extensively trained on these acquisition protocols to achieve the objective of enhancing the overall quality.

The instructions to perform an ordered image comprise the targeted structure or organ, the patient positioning instructions and the positioning instructions for the acquisition device (i.e. the acquisition geometry). All three are comprised in the acquisition protocol that comes with any ordered study. The acquisition geometry is defined by the direction of the imaging beam with respect to the patient position (also called angulation), the SID (source to image detector distance), the OID (object to image detector distance) and the beam collimation settings.

In some cases a plurality of series (or images) may be described in terms of an adapted acquisition protocol wherein for instance multiple projections may be requested to be performed.

When performing a study for a patient on an imaging modality, the operator will operate and configure the imaging modality via a modality console. The operator will receive the daily modality worklist at this console, which represents the list of all patients that require an imaging exam on that day. On the console the operator is able to select the correct patient by name or other criteria, so that all relevant personal information may be loaded in to the modality workstation at the moment that the exam is about to take place. This will allow to associate this data with any acquired image in the subsequently executed study. In most cases, this worklist information will comprise details about the ordered imaging study for the patient. This study may comprise the order for at least one medical image organised in at least one series. In the case of more than one ordered series for the same modality, the orders for the different images will be organized or grouped into a set of series.

In the context of this invention, the camera device that produces the reference image has to be understood as a regular digital visible light camera system, that is capable of capturing a color or grayscale 2D digital image. The camera system typically comprises a lens and a visible light matrix array detector, and captures a certain field-of-view in front of the lens. The patients' body -and more specifically the area of the body that comprises the targeted area- should be in-focus of the camera device. The digital images are outputted as digital files that can be processed by a computer system.

In the invention, the reference image is processed by a body part identification algorithm that essentially analyses and segments an incoming image. In a first instance, the algorithm determines the contours of the body. It subsequently segments the visible parts of the body in the image into different body parts. Further information about the body parts and the viewing angle of the camera onto the body part may also be produced. The algorithm may for instance segment and identify a chest in the image, and at the same time provide information that the view onto the chest in the image is a posterior view. Further measurements, such as for instance a distance from the center line from the chest may be provided and for instance encoded (by for instance a color difference) in a processed image.

As an example of such a body part identification algorithm, we refer to the DensePose project (http://densepose.org/, Authors: Riza Alp Guler, Natalia Neverova, Vasil Khalidov and lasonas Kokkinos).

The modality console will in most cases be equipped with a display monitor or screen that is able to display the modality worklist, and also the different series and images for the selected patient. When a certain patient record is selected from the worklist, the data for the patient in question will be loaded and his study details will be shown. The study may comprise one or more series of which the executions are defined by their acquisition protocols. These series may comprise in their own turn a number of images or acquisitions to be performed.

A common way to represent the different series or images to are to be executed by the operator for the study is by means of displaying a graphical representation for each item on the display. These graphical representations may be items in a flat or hierarchical list, or may be a set of grouped icons, ungrouped icons or thumbnail representations. The graphical representations of the ordered series or images may then be selected by the operator to let the system know which next image is going to be acquired. The selection may be performed for instance by means of a touchscreen or mouse control, and will be effected by a change of the visual appearance of the item, icon or thumbnail representation on the display. This change in appearance may for instance be the change in color or shading of the graphical representations.

As soon as the image is acquired, the graphical representations are removed from the display or may be grayed out to indicate that the series or image is successfully completed.

Certain systems will require the operator to select the series or image that will be executed next, such that the operator must explicitly for every acquisition make a selection of the image on the display. Performing this selection will allow the system to correctly associate the image data with a certain acquisition (ordered image). Alternatively, a system may force the operator to follow a predefined sequence of images to be acquired, which relieves the operator from making explicit selections for each upcoming acquisition, but requires him to follow a strict acquisition order. Both ways of working have their advantages and disadvantages.

The method of the invention however takes these disadvantages away, as the operator will not have to make explicit selections on the screen to select the desired order of the acquisitions, or to make explicit selections on the screen in case he wants to deviate from the pre-programmed or preconfigured order of acquisitions. The method allows the operator to maintain the freedom to alter the order of acquisitions at all times because the system and method of the invention will automatically detect and record which order has been chosen by the operator. The invention is thus advantageous in that it automatically detects which of the ordered image has been acquired after each acquisition step.

The embodiments of the systems and methods described herein may be implemented in hardware or software, or a combination of both. However, preferably, these embodiments are implemented in computer programs executing on programmable computers each comprising at least one module component which comprises at least one processor (e.g. a microprocessor), a data storage system (including volatile and non-volatile memory and/or storage elements), at least one input device, and at least one output device. For example and without limitation, the programmable computers (referred to here as computer systems) may be a personal computer, laptop, personal data assistant, and cellular telephone, smart-phone device, tablet computer, and/or wireless device. Program code is applied to input data to perform the functions described herein and generate output information. The output information is applied to one or more output devices, in known fashion.

Each program is preferably implemented in a high level procedural or object oriented programming and/or scripting language to communicate with a computer system. However, the programs can be implemented in assembly or machine language, if desired. In any case, the language may be a compiled or interpreted language. Each such computer program is preferably stored on a storage media or a device (e.g. ROM or magnetic diskette) readable by a general or special purpose programmable computer, for configuring and operating the computer when the storage media or device is read by the computer to perform the procedures described herein. The subject system may also be considered to be implemented as a computer-readable storage medium, configured with a computer program, where the storage medium so configured causes a computer to operate in a specific and predefined manner to perform the functions described herein.

Specific examples and preferred embodiments are set out in the dependent claims. Further advantages and embodiments of the present invention will become apparent from the following description and drawings.

### Brief description of drawings

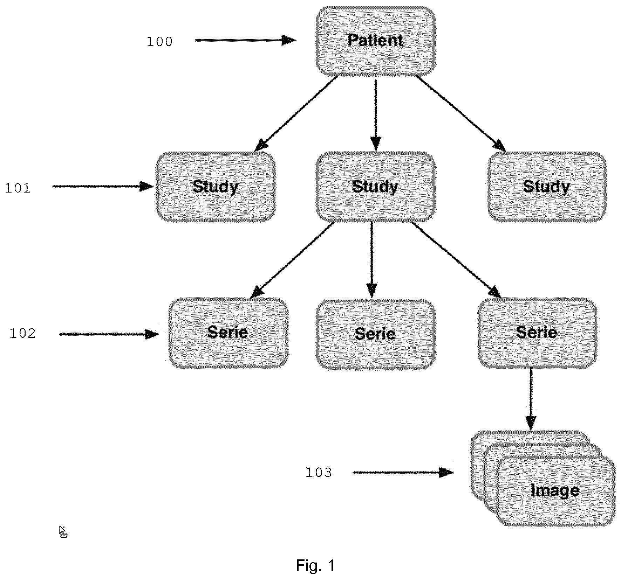

Fig. 1 gives an overview of the four hierarchical levels that are adhered to in the DICOM standard. [100] represents the highest level; the patient. A patient [100] may be attributed multiple studies [101]. A study may be attributed multiple series [102], which in its own turn may comprise multiple images [103].

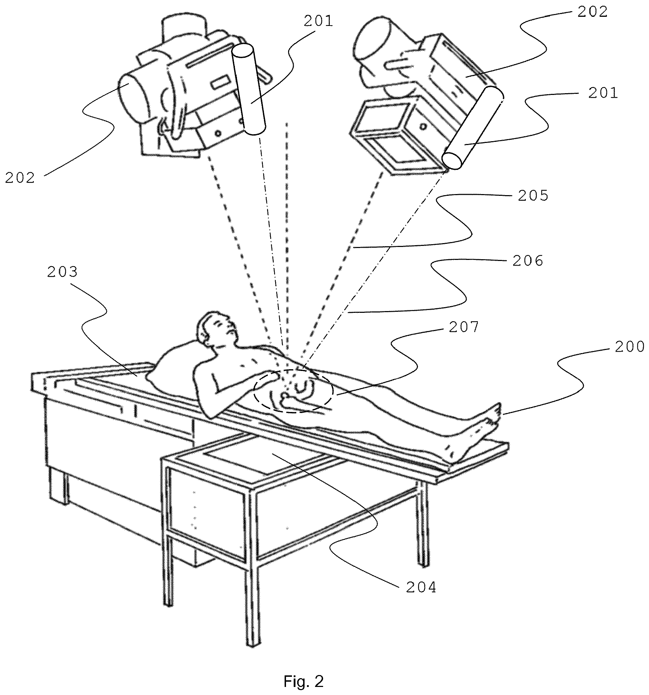

Fig. 2 depicts an imaging modality of the invention comprising a camera device [201] that is physically associated with the acquisition source [202]. The drawing further shown a patient [200] that is positioned on a positioning device [203] to bring the region of interest [207] of the patient in front of the imaging detector [204]. It is noted that the camera view direction [206] is in this case aligned to the beam direction of the imaging source [205].

### Description of embodiments

In the following detailed description, reference is made in sufficient detail to the above referenced drawings, allowing those skilled in the art to practice the embodiments explained below.

The method of the invention intends to improve the radiography acquisition workflow for an acquisition study for a patient on a radiography system in the sense that it allows the radiographer to freely perform the ordered image acquisitions in any order he desires to follow. As opposed to methods used in the state of the art that pre-define the acquisition order sequence, or that allow an operator to alter a pre-scheduled acquisition sequence, the method of the invention automatically detects which of the ordered acquisitions in the acquisition study is being performed. This automatic detection allows the system to determine the acquisition status of the ordered image acquisitions in the acquisition series, or study. As soon as an ordered image is acquired, the system will identify it amongst the other ordered images and will change its status accordingly in the order list.

The advantage of this method is that it relieves the operator from expressly having to follow a pre-determined acquisition order, and also that this method relieves the operator from having to perform certain operations on the acquisition console in case that he wants to alter this pre-determined acquisition order. The first advantage improves the flexibility for the operator to choose the acquisition order. The second advantage removes the need to perform any manual actions on the acquisition console in the case that the acquisition order (represented by the worklist) needs to be altered.

The imaging modality of the invention comprises an operator console that is configured to display for each patient a work list for all ordered and unprocessed images of the imaging study. The operator console therefore comprises a display device of some sort that may be a computer monitor or other digital display. The ordered images on the work list are represented on the display device as itemised elements represented by text items in a list, or as icons or thumbnails representing such an ordered item. The operator console is configured to display at least the unprocessed images in the study because these only need the attention of the operator. The already processed ordered images may be omitted from the displayed list, or may be shown in a different representation that would suggest that these items are already processed and therefore do not require the attention of the operator. The display of processed items may be implemented by displaying such processed ordered images in the worklist as grayed-out elements, or as grayscaled images, or alike.

The imaging modality of the invention also comprises an imaging source and detector combination that can produce the intended medical images. This combination may be for instance an X-ray source and a digital X-ray detector that is suitable for projection radiography. These components are mounted in a certain configuration allowing a patient to be positioned such that a medical image may be acquired of the targeted body part or structure, and by using the ordered projection. For an X-ray projection modality, the patient is positioned between an X-ray source and an X-ray detector.

In order to apply a certain projection for the acquisition on a patient, a specific acquisition geometry is chosen that is defined by a unique set of modality parameters, taking into consideration the particular patient positioning. A different patient positioning will require a different acquisition geometry to achieve the same projection.

The set of modality parameters comprise the modality component parameters involved in achieving the projection. The modality component parameters are defined by the degrees of freedom that the modality component supports. The acquisition geometry of the modality has thus to be understood as a combination of modality component parameters that define the geometric configuration of the modality that can perform the desired projection defined in the acquisition protocol. Depending on the nature of the modality, the modality parameters will vary depending on the types of components that make up the modality, and that determine the acquisition geometry. Some modalities will have a collimator suspension system that revolves isocentrically around an isocenter and of which the main modality parameters are defined in terms of angles, whereas a ceiling suspended collimator system may operate in terms of cartesian coordinates. In another example of a modality component, will the configuration parameters of a positioning table (height, lateral and longitudinal displacement of the table top) contribute to the geometric configuration of the modality.

The method of the invention intends to improve the radiography workflow, by automating the association of an acquired (or about to be acquired) image (that is characterised by a positioning situation of the patient and the modality, and information that is present in a reference image that is acquired by a camera device) with one of the ordered images in the study.

On one hand there is the list of ordered images in the radiography study that are defined by their particular acquisition protocols, and on the other hand there is an actual positioning situation of a patient that is captured in a visual image by a camera device, and the actual acquisition geometry of the modality that is available as digital readings of the modality parameters. The acquisition geometry of the modality is characterised by the digital readings of the different modality components that are available as digital data; these readings for instance provide information about the gantry angle, collimator angle, table height, table position, and alike. Whereas the positioning situation of the patient can be extracted from the reference image through the analysis by a body part identification algorithm. The position of the patient in combination with the acquisition geometry of the modality will define which body part or structure is targeted in the radiography study.

It is however important to note that an acquired image can only be associated with an ordered image from the worklist on condition that all information is captured when the patient and modality are in the intended position for the image acquisition. The collection of the information can thus be done briefly before the acquisition, briefly after the acquisition, and preferably during the acquisition. Alternatively, the above data may be acquired continuously and may be continuously evaluated, so to respond with an update of the displayed representations of the ordered images in the study in case that one of the intended ordered images is identified.

The improvement of the radiography workflow is thus achieved by automatically identifying the targeted structure on the patient and the applied projection for the acquisition, and matching this information with information in any of the acquisition protocols of the ordered images, such that an actual acquisition configuration (the actual acquisition configuration being the combination of the patient positioning and the modality configuration at a particular moment) can be associated with one of the ordered images in the study. When this information can be obtained, the system can compare this information with the acquisition protocols of the ordered images to determine which ordered image was executed at the time of the acquisition of the reference image. In case that such a comparison leads to a positive match, the actual acquisition configuration can be associated with the particular ordered image.

In order to achieve this objective, the method requires the acquisition of a reference image from a camera device that is positioned such that it comprises the presence of the (X-ray) beam entry area in the image and some additional image context allowing a body part algorithm to identify on which body part said beam entry area is located. The visible light camera device must have a clear view of the patient's body (or at least a part the patients' body) when acquiring the reference image, as the reference image will be used to identify the targeted body part or structure. The reference image is thus captured by a digital visual light camera covering a field of view that exceeds the size of the beam entry area on the patient. The reference image should at least cover the entire beam entry area, but also sufficient clinical context (i.e. complete views on the targeted body part and preferably its immediate surroundings) to allow the body part detection algorithm to properly function.

The camera device may be aligned with the beam direction of the acquisition source, or may even be physically associated with it, but this is not a requirement. Under all circumstances, must the camera device have the X-ray illuminated region (= beam entry region) into its field of view. The method will not work when the camera's view on this region is obstructed by an object, such as a table, positioning device or alike. The camera device will produce a digital image (preferably a color image) that subsequently has to be processed by an algorithm that identifies the body part or structure and the projection of the image.

A plurality of cameras may be applied that each are oriented into the direction of a possible detector position. One of the cameras may for instance be oriented towards a wallstand, while the other camera is oriented towards the X-ray positioning table, and this in order to ensure that the workflow improvement is applicable for all types of exposures that the modality system can handle.

The visible light camera device is preferably a regular photographic or video camera that produces images in the visible spectrum. The camera device is focussed on the skin surface of the patient, and has a field-of-view that has at least the same size as the beam of the acquisition source.

In a preferred embodiment, the camera device is a regular color video camera capable of producing a reliable reference image of the positioned patient with respect to the imaging device. Alternatively, a 3D or depth camera may be considered that produces so-called depth images of the patient.

In order to reduce the risk of obstruction of the camera view by other objects, the camera device may be aligned to the beam direction of the acquisition source.

Preferably, the field-of-view of the camera device is chosen considerably larger than the projected beam size on the patient, as it will produce an image that covers a larger area of the patient, compared to the region-of-interest alone. Selecting a larger area of the patient around the projected beam size (that is covering the region of interest) is advantageous for the identification of the region-of-interest captured by the reference image produced by the camera.

It is important to note that the reference image has to be acquired under the same patient positioning conditions and acquisition geometry as one of the ordered image acquisitions of the study. The easiest way to achieve this is to acquire both images at the same time, or at approximately the same time. The only aspect to observe is namely to ensure that neither the patient nor the imaging system would move between the acquisition of the visible and medical image. Alternatively, it would be conceivable to continuously evaluate the correspondence between the reference image and the patient positioning conditions and acquisition geometry of the ordered image acquisitions of the study.

In a next step, an algorithm identifies the location of the beam entry location (entering the surface of the patient) in the reference image. Depending on the location of the camera device with respect to the imaging modality, the determination of the beam entry location in the reference image will be straight forward or may require some additional calculations.

In case that the camera device is mounted on or in a fixed relation to the collimator of the X-ray tube, the beam entry location has a fixed relation to the field of view of the camera device for a particular location. In this case, the beam entry location will always appear in a same fixed location of the reference image. The field of view of the camera device may for instance be centered on the center of the X-ray beam (and thus on the beam entry location). In this case, it is easy to determine the beam entry location.

In another case where the camera device would be mounted on a fixed position somewhere in the X-ray room, the calculation of the location of the beam entry location in the reference image will require some more geometric considerations, as it will have to take the perspective of the camera into consideration. It is however possible to calculate the projection of the X-ray beam within the reference image using straightforward mathematics when the acquisition geometry of the X-ray modality is known.

In a next step, a body part identification algorithm identifies the body part on which the beam entry area is located. The result of this identification should correspond to a targeted structure in one of the ordered images, so that this body part can be compared with the targeted structures in all ordered images.

In order to do so, the body part algorithm typically identifies all body parts that are visible in the reference image. In this context, it is clear that when the reference image comprises more image context around the beam entry location, the body part identification will be performed more accurately, and the algorithm will be able to identify more body parts. But this comes at the cost of accuracy at which the beam entry location is represented in the reference image. The surface representing the beam entry location in the reference image will become smaller when more body context is brought within view of the camera device.

As soon as different body parts are being identified in the image, the body part associated with the beam entry location will be considered as the targeted organ or structure. Further distance information may provide a finer determination of the actual targeted structure.

The larger the image context around the region of interest is, the more detectable features are present in the reference image that may be identified by the body part identification algorithm. Body part detection algorithms such as disclosed in "Densepose: Dense human pose estimation in the wild", by Riza Alp Güler, Natalia Neverova, lasonas Kokkinos (Proceedings of the IEEE Conference on Computer Vision and Pattern Recognition, p. 7297-7306, 2018) are known in the art and are suitable for application in this method. Since generally recognition algorithms are known to respond better to clearly detectable features in the image. A larger image context around the region of interest will therefore ensure better performance of the body part identification algorithm.

Next to the identification of the targeted structure or organ, other detailed information regarding for instance the exact location of the beam entry area may be obtained from the body part detection algorithm. The algorithm may for instance return information regarding the exact beam entry location on the detected body part with respect to other reference structures visible in the image, such as for instance on which side (front, back, left, right) of this body part the beam entry location is located, at which distance it is located from the top of the identified body part, etc...

This information can then be attributed to a certain patient position when it is assumed that the location of the camera device is known. This patient position information together with the acquisition geometry (read out as digital data from the modality) provides the information about the projection that is used for the image acquisition.

All the above information (target structure, patient positioning and projection information) is then compared with the acquisition protocol data of all ordered images in the worklist. In case of a positive match, it is safe to assume that the actual acquisition in progress corresponds to the matching ordered image. Further variables can be additionally verified or compared to obtain confirmation about the correct match or the closest match. In case that the match would not be exact, the closest match may be proposed as the most suitable match while the operator may be alerted about the uncertainty of the absolute correctness of the proposal.

In case of a positive match of the body part and view position information in the visible reference image and the acquired image, the corresponding image order in the acquisition order list may be listed as completed, or may be removed from the order list. The image order will thus disappear from the order list, leaving only the outstanding ordered images on the list.

The positive match can then be used to change the status of the corresponding elements in the worklist. In case of a positive match, the element representing the ordered image in the worklist may be highlighted as "being processed", and may be for instance removed from the worklist after the actual acquisition was completed.

Further to this completion signal of the image, a number of quality control actions may be performed on the acquired image such as a check on the correct collimator settings, the exposure, the patient positioning or alike.

Alternative working detection algorithms may be conceived that are for instance based on neural networks. A neural network may be trained by feeding it with a dataset of training images that are tagged with the expected outcome data. The training is completed when the algorithm is successfully validated against a different set of similar images that are also tagged with the correct outcome data. The validation is successful when the body part detection on the validation set achieved a sufficient detection accuracy, which can be objectively assessed by evaluating the successful identifications compared to the total number of validation images.

In order to further improve the body part detection performance, additional input parameters, such as certain acquisition geometry parameters and/or patient positioning parameters, may be integrated into the body part detection algorithm. Examples of such parameters may be for instance the focal distance of the visible camera system (which determines the viewing angle), details about the patient's constitution (weight, body circumference, ...), or alike. These parameters may provide additional information to the algorithm as to which body parts may be excluded from detection when a certain acquisition geometry on a certain patient positioning.

## Claims

1. Method to improve a radiography acquisition workflow for an acquisition study for a patient on a radiography system, wherein said acquisition study comprises at least one ordered image in at least one image series for which an acquisition protocol is defined, said acquisition protocol comprising a target structure, a patient positioning instruction and an acquisition geometry, the method comprising the steps of:
- displaying said at least one ordered image as an item on an order list on a display means,
- positioning said patient on said radiography system according to said patient positioning instructions of said acquisition protocol of one of said ordered images,
- positioning said radiography system according to said acquisition geometry of said acquisition protocol of said one of said ordered images,
- capturing a reference image from a camera device, said reference image comprising a beam entry area on a surface of said patient defined by said acquisition protocol for said ordered image,
- identifying a body part and a location of said beam entry area on said body part on said reference image by means of a body part identification algorithm,
- matching said identified body part to a body part comprising said target structure defined in the acquisition protocol of any of the ordered images in said acquisition study, and
- matching said identified beam entry area to a beam entry area corresponding to an acquisition geometry and a patient positioning that is defined in the acquisition protocol of any of the ordered images in said acquisition study,
- displaying said ordered image as a marked item in said order list on said display means, in case of a positive match for said body part and said beam area.

2. Method according to Claim 1, wherein said marked item is removed from said order list as soon as the corresponding acquisition is completed.

3. Method according to Claim 1, wherein a view position from said camera device is aligned with a beam direction of an X-ray source of said radiography system, said beam direction being applied during one of said acquisitions of said ordered images.

4. Method according to Claim 3, wherein said camera device is attached to a collimator of said radiography system.

5. Method according to Claim 1, wherein said camera device is mounted on a fixed location in a room of said radiography system.

6. Method of Claim 1, wherein in case that said body part and said beam entry area cannot be matched to a body part and beam entry area of one of said ordered images in said order list, a user is alerted.

7. Method of Claim 1, wherein said body part detection algorithm is a neural network algorithm.

8. Method of Claim 1, wherein said display means is a computer monitor.

9. Method of Claim 1, wherein any marked ordered image is removed from said order list.

10. Method of Claim 1, wherein said order list is displayed as a set of thumbnail pictures that each graphically represent one of said ordered images.

## Patentansprüche

1. Verfahren zur Verbesserung eines Arbeitsablaufs einer Röntgenaufnahme für eine Aufnahmeanalyse für einen Patienten auf einem Radiographiesystem, wobei die Aufnahmeanalyse mindestens ein geordnetes Bild in mindestens einer Bilderserie, für die ein Aufnahmeprotokoll definiert ist, umfasst, wobei das Aufnahmeprotokoll eine Zielstruktur, eine Patientenpositionierungsanweisung und eine Aufnahmegeometrie umfasst, wobei das Verfahren die folgenden Schritte umfasst:
- Anzeigen des mindestens einen geordneten Bildes als ein Element auf einer Reihenfolgeliste auf einem Anzeigemittel,
- Positionieren des Patienten auf dem Radiographiesystem gemäß den Patientenpositionierungsanweisungen des Aufnahmeprotokolls eines der geordneten Bilder,
- Positionieren des Radiographiesystems gemäß der Aufnahmegeometrie des Aufnahmeprotokolls des einen der geordneten Bilder,
- Erfassung eines Referenzbildes aus einer Kameravorrichtung, wobei das Referenzbild einen durch das Aufnahmeprotokoll für das geordnete Bild definierten Strahleneingangsbereich auf einer Oberfläche des Patienten umfasst,
- Identifizierung eines Körperteils und einer Stelle des Strahleneingangsbereichs auf dem Körperteil auf dem Referenzbild mittels eines Körperteilidentifizierungsalgorithmus,
- Matching des identifizierten Körperteils mit einem Körperteil, der die im Aufnahmeprotokoll eines beliebigen der geordneten Bilder in der Aufnahmeanalyse definierte Zielstruktur umfasst, und
- Matching des identifizierten Strahleneingangsbereichs mit einem Strahleneingangsbereich in Übereinstimmung mit einer Aufnahmegeometrie und einer Patientenpositionierung, die im Aufnahmeprotokoll eines beliebigen der geordneten Bilder in der Aufnahmeanalyse definiert sind,
- Anzeigen des geordneten Bildes als markiertes Element in der Reihenfolgeliste auf dem Anzeigemittel, falls für den Körperteil und den Strahlenbereich eine positive Übereinstimmung erhalten wird.

2. Verfahren nach Anspruch 1, wobei das markierte Element sofort nach Vollendung der entsprechenden Erfassung aus der Reihenfolgeliste entfernt wird.

3. Verfahren nach Anspruch 1, wobei eine Ansichtsposition aus der Kameravorrichtung mit einer Strahlenrichtung einer Röntgenquelle des Radiographiesystems ausgerichtet wird, wobei die Strahlenrichtung während einer der Erfassungen der geordneten Bilder angewandt wird.

4. Verfahren nach Anspruch 3, wobei die Kameravorrichtung an einem Kollimator des Radiographiesystems befestigt ist.

5. Verfahren nach Anspruch 1, wobei die Kameravorrichtung an einer festen Stelle in einem Raum des Radiographiesystems befestigt ist.

6. Verfahren nach Anspruch 1, wobei, falls keine Übereinstimmung zwischen einerseits dem Körperteil und dem Strahleneingangsbereich und andererseits einem Körperteil und einem Strahleneingangsbereich eines der geordneten Bilder in der Reihenfolgeliste erhalten wird, ein Benutzer darauf hingewiesen wird.

7. Verfahren nach Anspruch 1, wobei der Körperteildetektionsalgorithmus ein Algorithmus eines neuronalen Netzwerks ist.

8. Verfahren nach Anspruch 1, wobei das Anzeigemittel ein Computermonitor ist.

9. Verfahren nach Anspruch 1, wobei ein beliebiges markiertes geordnetes Bild aus der Reihenfolgeliste entfernt wird.

10. Verfahren nach Anspruch 1, wobei die Reihenfolgeliste als Satz von Thumbnailbildern, die jeweils grafisch eines der geordneten Bilder darstellen, angezeigt wird.

## Revendications

1. Procédé pour améliorer un flux de travail d'une acquisition radiographique pour une analyse d'acquisition pour un patient sur un système radiographique, **caractérisé en ce que** ladite analyse d'acquisition comprend au moins une image ordonnée dans au moins une série d'images pour laquelle il est défini un protocole d'acquisition, ledit protocole d'acquisition comprenant une structure cible, une instruction de positionnement de patient et une géométrie d'acquisition, **caractérisé en ce que** le procédé comprend les étapes consistant à:
- afficher ladite au moins une image ordonnée comme un élément dans une liste séquentielle sur un moyen d'affichage,
- positionner ledit patient sur ledit système radiographique conformément auxdites instructions de positionnement de patient dudit protocole d'acquisition d'une des images ordonnées,
- positionner ledit système radiographique conformément à ladite géométrie d'acquisition dudit protocole d'acquisition de ladite une desdites images ordonnées,
- acquérir une image de référence d'un dispositif de caméra, ladite image de référence comprenant une zone d'entrée de rayonnement sur une surface dudit patient telle que définie par ledit protocole d'acquisition pour ladite image ordonnée,
- identifier une partie de corps et une position de ladite zone d'entrée de rayonnement sur ladite partie de corps sur ladite image de référence au moyen d'un algorithme d'identification de partie de corps,
- établir la correspondance entre ladite partie de corps identifiée et une partie de corps comprenant ladite structure cible définie dans le protocole d'acquisition d'une quelconque des images ordonnées dans l'analyse d'acquisition, et
- établir la correspondance entre ladite zone d'entrée de rayonnement identifiée et une zone d'entrée de rayonnement correspondant à une géométrie d'acquisition et un positionnement de patient tels que définis dans le protocole d'acquisition d'une quelconque des images ordonnées dans l'analyse d'acquisition,
- afficher ladite image ordonnée comme élément marqué dans ladite liste séquentielle sur ledit moyen d'affichage dans le cas où il est établi une correspondance pour ladite partie de corps et ladite zone de rayonnement.

2. Procédé selon la revendication 1, **caractérisé en ce que** ledit élément marqué est éliminé de ladite liste séquentielle immédiatement après la fin de l'acquisition correspondante.

3. Procédé selon la revendication 1, **caractérisé en ce qu'**une position de vue dudit dispositif de caméra est alignée avec une direction de rayonnement d'une source de rayons X dudit système radiographique, ladite direction de rayonnement étant appliquée lors d'une desdites acquisitions desdites images ordonnées.

4. Procédé selon la revendication 3, **caractérisé en ce que** ledit dispositif de caméra est attaché à un collimateur dudit système radiographique.

5. Procédé selon la revendication 1, **caractérisé en ce que** ledit dispositif de caméra est monté à une position fixe dans un espace dudit système radiographique.

6. Procédé selon la revendication 1, **caractérisé en ce qu'**un utilisateur reçoit un avertissement dans le cas où il n'est établi aucune correspondance entre d'une part ladite partie de corps et ladite zone d'entrée de rayonnement et d'autre part une partie de corps et une zone d'entrée de rayonnement d'une desdites images ordonnées dans ladite liste séquentielle.

7. Procédé selon la revendication 1, **caractérisé en ce que** l'algorithme de détection de partie de corps est un algorithme de réseau neuronal.

8. Procédé selon la revendication 1, **caractérisé en ce que** ledit moyen d'affichage est un moniteur d'ordinateur.

9. Procédé selon la revendication 1, **caractérisé en ce qu'**une quelconque image ordonnée marquée est éliminée de ladite liste séquentielle.

10. Procédé selon la revendication 1, **caractérisé en ce que** ladite liste séquentielle est affichée comme un ensemble de vignettes qui représentent chacune de manière graphique l'une desdites images ordonnées.
